## Europäisches Patentamt

## European Patent Office

## Office européen des brevets

(19)

(11) Veröffentlichungsnummer: **0 033 881**
**B1**

(12) **EUROPÄISCHE PATENTSCHRIFT**

(45) Veröffentlichungstag der Patentschrift:
**09.11.83**

(51) Int. Cl.³: **C 07 C 69/54,** C 07 C 67/343

(21) Anmeldenummer: **81100530.5**

(22) Anmeldetag: **24.01.81**

(54) **Verfahren zur Herstellung von Methylmethacrylat durch Umsetzung von Methylpropionat und Methanol.**

(30) Priorität: **07.02.80 DE 3004467**

(43) Veröffentlichungstag der Anmeldung:
**19.08.81 Patentblatt 81/33**

(45) Bekanntmachung des Hinweises auf die Patenterteilung:
**09.11.83 Patentblatt 83/45**

(84) Benannte Vertragsstaaten:
**BE DE FR GB IT NL**

(56) Entgegenhaltungen:
**DE - A - 1 905 763**
**DE - A - 2 525 174**
**GB - A - 1 428 277**

(73) Patentinhaber: **BASF Aktiengesellschaft,**
**Carl-Bosch-Strasse 38, D-6700 Ludwigshafen (DE)**

(72) Erfinder: **Merger, Franz, Dr., Max-Slevogt-Strasse 25,**
**D-6710 Frankenthal (DE)**
Erfinder: **Fouquet, Gerd, Dr., Maxburgstrasse 2,**
**D-6730 Neustadt (DE)**

BUNDESDRUCKEREI BERLIN

## Verfahren zur Herstellung von Methylmethacrylat
### durch Umsetzung von Methylpropionat und Methanol

Aus der US-PS 3 014 958 ist es bekannt, Formaldehyd in wäßriger Lösung mit Methylpropionat im molaren Verhältnis 1 : 1,5 bis 1 : 20 in Gegenwart von Dehydratisierkatalysatoren bei 225 bis 450°C umzusetzen. Dabei wird in Gegenwart von mindestens 7 Gew.-% Methylmethacrylat unter Zusatz von Methanol gearbeitet. Bei Verwendung eines Katalysators der allgemeinen Formel $K_2O \cdot MgO \cdot Fe_2O_3$ wird eine Ausbeute von 84%, bezogen auf Formaldehyd, erzielt, wenn man bei einem molaren Verhältnis von Methylpropionat zu Formaldehyd zu Wasser zu Methylmethacrylat von 10 : 1 : 1 : 0,8 in Gegenwart geringer Mengen Methanol arbeitet. Bei Verwendung von Calciumphosphat, Calciumsulfat, Aluminiumphosphat oder Magnesiumphosphat werden niedrigere Ausbeuten erhalten.

Aus der britischen Patentschrift 1 447 669 ist es bekannt, $\alpha,\beta$-äthylenisch ungesättigte Säuren oder deren Ester herzustellen, indem man Formaldehyd mit der entsprechenden Alkansäure bzw. ihren Estern in Gegenwart von pyrogener Kieselsäure als Katalysator umsetzt, die mit bis zu 10% ihres Gewichts pyrogenem Zirkondioxid vermischt sein kann und eine Gesamtoberfläche von 150 bis 300 m² je g, eine Porosität von 3 bis 15 cm³ je g und eine Porengrößenverteilung derart aufweist, daß mindestens 50% der Poren einen Durchmesser über 10 000 Å und weniger als 30% der Poren einen Durchmesser unter 1000 Å haben. Der verwendete Katalysator soll mit einem basischen Stoff aktiviert und dann calciniert sein. Die Reaktion wird bei 330 bis 390°C durchgeführt, wobei das molare Verhältnis von Alkansäure bzw. deren Ester zu Formaldehyd im Bereich von 0,3 : 1 bis 3,0 : 1 liegt. Als basisches Aktivierungsmittel kommt vorzugsweise Natriumhydroxid, Kaliumhydroxid, Caesiumhydroxyd, Calciumhydroxyd und Calciumcarbonat in Frage. Bei dem einzigen Beispiel dieser Patentschrift, das die Umsetzung von Methylpropionat betrifft wird bei einem molaren Verhältnis von Methylpropionat zu Formaldehyd zu Wasser zu Methanol von 20 : 20 : 59 : 1 bei 370°C gearbeitet und ein Umsatz von 25% erzielt, wobei ein Gemisch von Methacrylsäure und Methylmethacrylat in einer Ausbeute von zusammen 63 bzw. 44%, bezogen auf Formaldehyd bzw. Methylpropionat erhalten werden. Ein Nachteil dieses Verfahrens ist, daß hierbei 40% des eingesetzten Methylpropionats zu Propionsäure verseift wird.

Aus der britischen Patentschrift 1 428 277 ist bekannt, Methylmethacrylat aus Methylpropionat, Formaldehyd, Wasser und ggf. Methanol in Gegenwart von Verbindungen der ersten Hauptgruppe des periodischen Systems bei einer Reaktionstemperatur von 400 bis 600°C herzustellen. Die Katalysatoren sollen eine spezifische Oberfläche von 350 bis 1000 m² je g haben und das molare Verhältnis von Wasser zu Formaldehyd im Bereich von 0,01 : 1 bis 10 : 1 liegen. Im Beispiel 1 wird bei einem molaren Verhältnis von Methylpropionat zu Formaldehyd zu Wasser zu Methanol von 4,5 : 1 : 5,3 : 6,7 ein Formaldehydumsatz von 67% und eine Ausbeute an Methylmethacrylat von 92%, bezogen auf den Formaldehyd erhalten. Als Katalysator dient dabei Silicagel einer spezifischen Oberfläche von 600 m² je g, das bei 500°C calciniert und mit KOH aktiviert ist. Eine Verseifung des Methylpropionat zu Propionsäure wurde dabei nicht beschrieben. Ohne Zusatz von Wasser erfolgt jedoch bei diesem Verfahren praktisch keine Kondensation zu Methylmethacrylat (vgl. Beispiel 4, Run 3).

Aus der DE-OS 2 525 174 ist es bekannt, Methanol in Gegenwart von Kupfer, Zink und Schwefel als Katalysatorkomponenten bei 500 bis 750°C zu dehydrieren. Man erhält dabei eine methanolische Formaldehydlösung, die zu 30 bis 85 Gew.-% aus Formaldehyd besteht.

Aus der DE-OS 2 627 421 ist es bekannt, Methanol in Anwesenheit eines Katalysators, der Kupfer, Zink und Selen als Komponenten enthält, bei 500 bis 750°C zu dehydrieren. Der Methanolumsatz beträgt bis zu 78%, die Selektivität an Formaldehyd bis zu 90%.

Schließlich ist es aus der DE-OS 1 905 763 bekannt, Methylacrylat durch Umsetzen von Methylacetat und Methanol in Gegenwart von Sauerstoff und Silber oder Kupfer auf einem Träger aus einem Aluminiumsilikat bei Temperaturen von 350−500°C herzustellen. Durch die Mitverwendung von Sauerstoff wird dabei die Bildung von molekularem Wasserstoff vermieden.

Es wurde nun gefunden, daß man Methylmethacrylat durch Umsetzen von Methylpropionat bei 200−550°C in Gegenwart von Methanol an Katalysatoren herstellen kann, indem man Methylpropionat und Methanol an einem Kupfer und Zink sowie Tellur und/oder Selen und/oder Schwefel enthaltenden oxidischen Katalysator umsetzt. Der Katalysator wirkt kondensierend und dehydrierend und ermöglicht es, Methanol bei 200−550°C zu dehydrieren, wobei unter diesen Bedingungen eine Hydrierung des gebildeten Methylmethacrylats durch den bei der Dehydrierung gebildeten Wasserstoff höchstens in untergeordnetem Ausmaß stattfindet. Bei dem Verfahren arbeitet man im allgemeinen ohne Zusatz von gasförmigem Sauerstoff zum Reaktionsgemisch, doch stören geringe Spuren Sauerstoff, wie sie als Verunreinigung der Ausgangsstoffe ggf. eingeschleppt werden, im allgemeinen nicht. In den besonders geeigneten kondensierend und dehydrierend wirkenden Katalysatoren beträgt das atomare Verhältnis von Kupfer zu Zink zu Teller und/oder Selen im allgemeinen 1 zu 0,01 bis 0,5 zu 0,001 bis 0,3, vorzugsweise 1 zu 0,05 bis 0,4 zu 0,005 bis 0,2. Derartige Katalysatoren können z. B. hergestellt werden, indem man Kupferoxid mit Zinkoxid und Tellurdioxid (und/oder Selendioxid und/oder Ammoniumsulfat) unter Zugabe von Wasser verknetet, das Gemisch bei 130°C trocknet und dann zu Pillen verpreßt. Dabei kann auch Trägermaterial zugemischt werden.

Außerdem ist es möglich, hierbei weitere, kondensierend wirkende Bestandteile, insbesondere mit Alkali aktiviertes Siliciumdioxid zusammen mit den oxidischen Katalysator-Bestandteilen unter Zusatz geringer Mengen Wasser zu vermischen, zu trocknen und zu Pillen zu verpressen.

Anstelle von mit Alkali aktiviertem Siliciumdioxid können auch andere kondensierend wirkende Katalysatoren mitverwendet werden, wie sie beispielsweise in den britischen Patentschriften 1 447 669 und 1 428 277 sowie in der US-PS 3 014 958 beschrieben sind. Genannt seien beispielhaft die Phosphate, Silikate und Oxide vor allem des Aluminiums sowie ferner des Magnesiums, Kalziums, Titans und Silikons, die ggf. mit Oxiden und/oder Hydroxiden von Alkali- und/oder Erdalkalimetallen insbesondere mit KOH, NaOH oder CsOH sowie ferner CaO, Ca(OH)$_2$, MgO oder Mg(OH)$_2$ modifiziert sind. Für das Modifizieren des Siliciumdioxids geeignete Alkalioxide sind besonders K$_2$O, Na$_2$O oder Cs$_2$O. Mit Alkalioxid modifiziertes Siliciumdioxid kann mit Vorteil auch hochdispere Kieselsäure, gewonnen durch Flammenhydrolyse flüchtiger Siliciumverbindungen (Gesamtoberfläche 100 bis 400 mm$^2$/g) hergestellt werden. Das Aufbringen des Modifizierungsmittels kann durch Tränken erfolgen.

In den oxidischen Katalysatoren der genannten Art wird das Kupferoxid beim Einsatz ganz oder teilweise durch Wasserstoff, der bei der Dehydrierung des Methanols entsteht, zu metallischem Kupfer reduziert. In manchen Fällen ist es von Vorteil, den Katalysator vor seinem Einsatz beispielsweise mit gasförmigem Wasserstoff bei 200 bis 600°C zu reduzieren.

Die Katalysatoren können bei dem Verfahren in den Reaktionsgefäßen, z. B. Röhrenreaktoren, fest angeordnet sein. Das neue Verfahren kann jedoch auch in der Wirbelschicht durchgeführt werden. Die Reaktionstemperatur beträgt im allgemeinen 200 bis 550°C, vorzugsweise 250 bis 500°C. Das molare Verhältnis von Methylpropionat zu Methanol beträgt meist 10 zu 1 bis 1 zu 10, vorzugsweise 5 zu 1 bis 1 zu 5, Wasser kann bei dem Verfahren zugegen sein, ist jedoch nicht erforderlich. Die Kontaktzeit (definiert als: g (Edukte), g$^{-1}$ (Katalysator), h$^{-1}$ (Reaktionsdauer) liegt meist im Bereich von 10 bis 0,05, vorzugsweise von 5 bis 0,05.

Das Reaktionsgemisch kann in üblicher Weise aufgearbeitet und nicht umgesetztes Ausgangsmaterial der Reaktion erneut zugeführt werden.

Die Feststellung, daß die erfindungsgemäßen Katalysatoren zur Dehydrierung vom Methanol zu Formaldehyd geeignet sind, beinhaltet keinesfalls eine Aussage über welche Stufen bzw. Zwischenstufen das erfindungsgemäße Verfahren (Dehydrierung und Kondensation) verläuft.

Es ist überraschend, daß die Dehydrierung von Methanol und die Umsetzung mit Methylpropionat zu Methylmethacrylat in einem »Eintopf-Verfahren« durchgeführt werden kann, da nicht zu erwarten war, daß einmal der bei der Dehydrierung des Methanols freigesetzte Wasserstoff in Gegenwart der Katalysatoren nicht zu wesentlicher Hydrierung des Methylmethacrylates führt und zum anderen eine Störung der Dehydrierung des Methanols durch Methylmethacrylat nicht erfolgt. Die bevorzugten dehydrierend wirkenden Katalysatoren, die Kupfer, Zink und Tellur enthalten, sind zudem neu. Die Kupfer, Zink und Tellur enthaltenden Katalysatoren sind deshalb besonders geeignet, da sie im Gegensatz zu den bekannten Katalysatoren, die Kupfer, Zink und Schwefel oder Selen enthalten und während der Reaktion an Schwefel oder Selen verarmen, überraschenderweise kein Tellur verlieren.

Es ist ein Vorteil des neuen Verfahrens, daß es die Herstellung von Methylmethacrylat aus Methylpropionat in besonders einfacher und wirtschaftlicher Weise ermöglicht.

Für die im folgenden beschriebenen Beispiele wird jeweils wie folgt gearbeitet:

Eine Lösung von Methylpropionat in Methanol wird in einem mit Raschigringen gefüllten Quarzrohr von 20 cm Länge und 3 cm Innendurchmesser verdampft und die gasförmigen Reaktionskomponenten dann in den mit den Katalysatoren gefüllten Reaktor geleitet. Der Reaktor besteht aus einem Quarzrohr, das in einer Länge von 30 cm beheizbar ist und einen Innendurchmesser von 3 cm hat. Das den Reaktor verlassende Reaktionsgemisch wird gaschromatographisch untersucht. Die in den folgenden Beispielen angegebenen Selektivitäten wurden nach folgenden Gleichungen bestimmt:

$$\text{Umsatz (\%)} = \frac{\text{umgesetztes Methylpropionat in Mol}}{\text{zugeführtes Methylpropionat in Mol}} \times 100,$$

$$\text{Selektivität (\%)} = \frac{\text{gebildetes Methylmethacrylat in Mol}}{\text{umgesetztes Methylpropionat in Mol}} \times 100.$$

Beispiel 1

(a) Herstellung des Katalysators
   85,8 g Kupfer(II)oxid, 10 g Zinkoxid und 4,2 g Tellurdioxid werden mit 50 g Wasser gemischt und das Gemisch bei 130°C getrocknet. Das getrocknete Oxidgemisch wird zu Pillen eines Durchmessers von 3 mm und einer Höhe von 3 mm verpreßt.
(b) 100 g hochdisperse Kieselsäure (HDK-N-20 der Firma Wacker) werden in 1000 cm$^3$ einer 0,1%igen wäßrigen Kaliumhydroxid-Lösung suspendiert. Man dampft dann das Wasser in einem Rotationsverdampfer ab und trocknet den Rückstand 2 Stunden bei 130°C. Das getrocknete, mit KOH aktivierte SiO₂ wird zu Pillen von 3 mm Durchmesser und 3 mm Höhe verpreßt und anschließend bei 450°C 4 h calciniert.

(c) Umsetzung

30 cm³ (= 65,1 g) des Katalysators nach (a) und 150 cm³ (= 87,3 g) des Katalysators nach (b) werden in Schichten, beginnend mit 10 cm³ des Katalysators nach (a) und 50 cm³ des Katalysators nach (b) in den Reaktor eingefüllt. Es wird dann auf 420°C aufgeheizt und ein Gemisch von stündlich 7,7 g Methylpropionat und 13,9 g Methanol über den Katalysator geleitet. Dabei wird Methylpropionat mit einer Selektivität von 81,4% in Methylmethacrylat umgewandelt. Der Umsatz beträgt 13,2%. Methylisobutyrat wird mit einer Selektivität von 4,9% gebildet.

## Beispiel 2

Man arbeitet wie in Beispiel 1c) angegeben, leitet aber stündlich ein Gemisch von 89 g Methylpropionat und 80,8 g Methanol durch den Reaktor. Dabei wird Methylpropionat mit einer Selektivität von 76% in Methylmethacrylat umgewandelt und Methylisobutyrat mit einer Selektivität von 3,4% gebildet.

## Beispiel 3

(a) Herstellung des Katalysators

78,1 g Kupfer(II)oxid, 10,9 g Zinkoxid und 10,9 g Selendioxid werden mit 50 g Wasser verknetet und das Gemisch bei 130°C getrocknet. Das trockene Gemisch wird zu Pillen (3 mm × 3 mm) verpreßt.

(b) Umsetzung 60 ml (= 137 g) des Katalysators füllt man in den Reaktor und leitet bei 450°C je Stunde ein Gemisch aus 17,6 g Methylpropionat und 32 g Methanol über den Katalysator. Dabei wird Methylpropionat mit einer Selektivität von 46,1% zu Methylmethacrylat umgewandelt, der Umsatz betrug 5%. Methylisobutyrat wird mit einer Selektivität von 9,3% gebildet.

## Beispiel 4

(a) Herstellung des Katalysators

85,8 g Kupfer(II)oxid, 10 g Zinkoxid und 4,2 g Tellurdioxid werden mit 50 g Wasser verknetet, das Gemisch bei 130°C getrocknet und dann zu Pillen (3 mm × 3 mm) verpreßt.

(b) 100 g hochdisperse Kieselsäure (HDK-N-20 der Firma Wacker) werden in 1000 cm³ 0,1%iger wäßriger Kaliumhydroxidlösung suspendiert und dann das Wasser in einem Rotationsverdampfer von der Suspension abdestilliert. Der Rückstand wird 2 Stunden bei 130°C getrocknet und 4 Stunden bei 450°C getempert und schließlich zu einem Granulat einer Korngröße von 2 bis 3 mm gebrochen. Das Schüttgewicht des Katalysators beträgt 0,3 g je cm³.

(c) Umsetzung

30 cm³ (= 65 g) des Katalysators (a) und 150 cm³ (= 45,9 g) des Katalysators (b) werden, wie in Beispiel 1 angegeben, in dem Reaktor angeordnet. Man heizt dann auf 420°C auf und leitet je Stunde ein Gemisch aus 55,3 g Methylpropionat und 20,1 g Methanol durch den Reaktor. Dabei beträgt der Umsatz des Methylpropionats 5,8%, wobei Methylmethacrylat mit einer Selektivität von 85,6% gebildet wird. Methylisobutyrat erhält man mit einer Selektivität von 4,3%.

## Beispiel 5

Man arbeitet wie in Beispiel 4 (c) angegeben, leitet aber je Stunde 18,3 g Methanol und 20,1 g Methylpropionat durch den Reaktor. Der Umsatz an Methylpropionat beträgt dann 14,6%, die Selektivität 76,0%. Methylisobutyrat erhält man mit einer Selektivität von 7,3%.

## Beispiel 6

30 cm³ (= 65 g) des Katalysators (a) von Beispiel 4 und 145 cm³ (= 45,9 g) des Katalysators (b) von Beispiel 4 werden gemischt und in den Reaktor gefüllt. Man heizt auf 420°C und leitet je Stunde ein

**0 033 881**

Gemisch aus 8,5 g Methanol und 9,4 g Methylpropionat durch den Reaktor. Dabei beträgt der Umsatz an Methylpropionat 14%, die Selektivität an Methylmethacrylat 78,3%. Methylisobutyrat wird mit einer Selektivität von 5,9% gebildet.

### Beispiel 7

8 cm³ (= 18,3 g Katalysator-Pillen von Beispiel 3 (a) und 45 cm³ des in Beispiel 1 unter (b) angegebenen Katalysators werden vermischt und in den Reaktor eingebracht. Nach dem Aufheizen auf 420° C leitet man stündlich ein Gemisch von 7,5 g Methylpropionat und 8,3 g Methanol durch den Reaktor. Dabei wird das Methylpropionat zu 14,4% mit einer Selektivität von 49,8% in Methylmethacrylat umgewandelt. Methylisobutyrat erhält man mit einer Selektivität von 8,7%.

### Beispiel 8

30 cm³ (= 65 g) des Katalysators (a) von Beispiel 4 werden mit 150 cm³ (= 98,5 g) Aluminiumoxid (3-mm-Tabletten) vermischt und in den Reaktor gefüllt. Man heizt auf 400° C auf und leitet je Stunde ein Gemisch aus 26,8 g Methylpropionat und 24,3 g Methanol durch den Reaktor. Dabei beträgt der Umsatz des Methylpropionats 11,0%, wobei Methylmethacrylat mit einer Selektivität von 8,2% gebildet wird. Methylisobutyrat erhält man mit einer Selektivität von 6,4%.

### Beispiel 9

30 cm³ (= 65 g) des Katalysators (a) von Beispiel 4 werden mit 150 cm³ (= 98,5 g) Titandioxid (1,5 mm Stränge) vermischt und in den Reaktor gefüllt. Nach dem Aufheizen auf 410° C leitet man stündlich ein Gemisch aus 24,1 g Methylpropionat und 23,2 g Methanol durch den Reaktor. Dabei beträgt der Umsatz des Methylpropionats 13,6%, wobei Methylmethacrylat mit einer Selektivität von 50,1% gebildet wird. Methylisobutyrat erhält man mit einer Selektivität von 3,5%.

### Beispiel 10

30 cm³ (= 65 g) des Katalysators (a) von Beispiel 4 und 150 cm³ (= 117,5 g) Magnesiumoxid (3-mm-Stränge) werden, wie in Beispiel 1 beschrieben, in den Reaktor angeordnet. Man heizt dann auf 350° C und leitet stündlich ein Gemisch aus 26,8 g Methylpropionat und 24,3 g Methanol durch den Reaktor. Dabei beträgt der Umsatz des Methylpropionats 6,3%, wobei Methylmethacrylat mit einer Selektivität von 8,2% gebildet wird. Methylisobutyrat erhält man mit einer Selektivität von 16,2%.

### Beispiel 11

30 cm³ (= 65 g) des Katalysators (a) von Beispiel 4 und 150 ml (= 73,6 g) Aluminiumphosphat (1,5-mm-Stränge) dotiert mit 5,9% Kalium (als Kaliumpropionat eingeknetet) werden, wie in Beispiel 1 beschrieben, in dem Reaktor angeordnet. Man heizt auf 300° C und leitet stündlich 20,1 g Methylpropionat und 18,3 g Methanol durch den Reaktor. Der Umsatz an Methylpropionat beträgt dann 17%, wobei Methylmethacrylat mit einer Selektivität von 14,1% gebildet wird. Methylisobutyrat erhält man mit einer Selektivität von 7,6%.

## Patentanspruch

Verfahren zur Herstellung von Methylmethacrylat aus Methylpropionat bei 200 – 550° C in Gegenwart von Methanol an Katalysatoren, dadurch gekennzeichnet, daß man Methylpropionat und Methanol an einem Kupfer und Zink sowie Tellur und/oder Selen und/oder Schwefel enthaltenden oxidischen Katalysator umsetzt.

## Claim

A process for the preparation of methyl methacrylate from methyl propionate and methanol at 200 – 550° C in the presence of a catalyst, wherein methyl propionate and methanol are reacted over an oxide catalyst containing copper and zinc as well as tellurium and/or selenium and/or sulphur.

5

## Revendication

Procédé de préparation du méthacrylate de méthyle à partir du propionate de méthyle entre 200 et 550°C en présence de méthanol et d'un catalyseur, caractérisé en ce que l'on fait réagir le propionate de méthyle et le méthanol en présence d'un catalyseur d'oxydation, contenant du cuivre et du zinc, ainsi que du tellure et(ou) du sélénium et(ou) du soufre.